# EUROPEAN PATENT APPLICATION

(11) **EP 4 834 256 A1**
(43) Date of publication of application: **04.11.2026**
(21) Application number: 25172905.9
(22) Date of filing: 28.04.2025
(51) Int. Cl.: A61B 17/34, A61B 17/32

(54) **IMPLANTATION TOOL FOR IMPLANTING A MEDICAL IMPLANT SUBCUTANEOUSLY**

(71) Applicant: BIOTRONIK SE & Co. KG, 12359 Berlin (DE)
(72) Inventor: RODRIGUEZ, Valerie, Banks, 97106 (US); AUSTIN, Eric, Portland, 97221 (US); MELIUS, Matthew, Milwauki, 97267 (US)
(74) Representative: Biotronik Corporate Services SE

(57) **Abstract**

An implantation tool for implanting a medical implant subcutaneously, the implantation tool comprising:
- a handle extending along a longitudinal direction, with a through-hole, extending along the longitudinal direction, and at least one first and second groove extending on a surface of the handle facing the through-hole;
- a bottom clamshell slidably mounted in said first groove and a top clamshell slidably mounted in said second groove, said grooves are formed to define a carrying configuration, in which said clamshells protrude from the handle to hold the medical implant, and to assume to retract said clamshells into a retracted configuration, whereby said clamshells are shifted away from each other for releasing the medical implant from the implantation tool; and
- an operating member engaged with said clamshells whereby via a rotation of said operating member relative to said handle said operating member is switchable between a locked configuration and an unlocked configuration, to block sliding of said clamshells along said grooves in the locked configuration and to allow sliding of said clamshells via the operating member in the unlocked configuration.

## Description

The proposed solution relates to an implantation tool for implanting a medical implant subcutaneously, particularly for implanting an implantable cardiac monitoring device.

Such an implantable cardiac monitoring device is configured to monitor signals from the heart of the patient, particularly ECG signals in order to be able to, say, assess the condition of the heart, particularly heart failure (HF) and cardiac vascular diseases (CVD). Such implantable cardiac monitors (ICM) are becoming an important part of cardiac medical care. The goal of implanting an ICM is to provide clinicians and patients with biometric data that can be used to make informed decisions about care needs. Ideally, the insertion of this device will be perceived as a simple, quick, and easy outpatient procedure that minimizes surgical time and patient discomfort.

Regarding such devices, surgical tools have been developed that emphasize the "injectability" of such devices by employing syringe-like features (e.g., plungers), in order to achieve a relatively fast implantation procedure, minimizing surgical time and patient discomfort.

For example, U.S. Patent No. 11,266,440 B2 discloses a implantation tool that allows a fast and easy subcutaneous implantation of a medical device.

However, there is still room to make implantation easier and faster, and to reduce surgical time and patient discomfort.

Based on the above, it's an objective of the proposed solution to provide an implantation tool improving the above-mentioned aspects.

This objective is solved with an implantation tool for implanting a medical implant subcutaneously according to claim 1. Accordingly, the proposed implantation tool comprises at least:
- a handle extending along a longitudinal axis, said handle being formed with a through-hole extending along the longitudinal axis, at least a first groove and a second groove, said grooves extending on a surface of the handle facing the through-hole;
- a bottom clamshell slidably mounted in said first groove and a top clamshell slidably mounted in said second groove, said grooves are formed to define a carrying configuration of said clamshells, in which said clamshells protrude from the handle to hold the medical implant abutting an end of the handle, and to assume to retract said clamshells from said carrying configuration toward the handle into a retracted configuration, whereby said clamshells shifted away from each other along a direction transverse to the longitudinal direct for releasing the medical implant from the implantation tool; and
- an operating member engaged with said clamshells whereby via a rotation of said operating member relative to said handle said operating member is switchable between a locked configuration and an unlocked configuration, said operating member is configured to block sliding of said clamshells along said grooves in the locked configuration and to allow sliding of said clamshells via the operating member in the unlocked configuration.

The tool design works as follows: after a small incision is made, an implant held in place by the two clamshells is inserted under the skin, forming a pocket; after insertion, the tool handle is held in place while the operating member is rotated to unlock the tool and subsequently is drawn back, retracting the clamshells into the retracted configuration while maintaining the implant's position abutting the handle. Thereby, due to a form of the grooves the clamshells are actively spaced apart, i.e. opened transverse to the longitudinal direction (transverse direction), while retracted along the longitudinal direction. Accordingly, the clamshells are moved with a superimposed movement along the longitudinal direction on the one hand and transverse to the longitudinal direction on the other hand. This superimposed movement reduces friction of the clamshells while sliding relative to the implant which is held in place by abutting the handle on a proximal end of the handle facing the medical implant. This reduces the force needed for retracting the operating member, as well as vibrations of the clamshells and/or movement of the handle which can improve the patient's perception of the releasing process.

According to an embodiment of the proposed solution at least one of the first and second groove comprise two parallel segments spaced apart from each other along the transverse direction. This arrangement of parallel segments can enable a movement of the clamshell engaged with the at least one groove along the transverse direction when slid from the one of the two parallel segments to the other of the two segments. Accordingly, the top and the bottom clamshell can be spaced apart from each other by sliding one of the clamshells along the groove from one of the parallel segments into the other of the parallel segments. Thus, via sliding the clamshell or both clamshells, the clamshells can be opened along the transverse direction in order to release a medical implant which can be hold in between both clamshells in the carrying configuration. The medical implant may also be called implantable medical device (IMD).

According to an embodiment of the proposed solution, the two parallel segments can be connected by an angled keyway along which the clamshell engaged with the at least one groove can slide from the first of the parallel segments into the second of the parallel segments. Thus, the keyway can extend along the longitudinal direction and along the transverse direction enclosing an angle with the longitudinal direction (keyway angle). The connection of both parallel segments via the angled keyway can enable a smooth transition of the clamshell engaged with the at least one groove from the first of the parallel segments into the second of the parallel segments.

According to an embodiment of the proposed solution, the at least one first groove and the at least one second groove both comprise two parallel segments spaced apart from each other along a direction transverse to the longitudinal direction. Thus, the bottom clamshell and the top clamshell can be moved along the transverse direction while retracting along the longitudinal direction. Thereby, an amount each of the clamshells has to be moved along the transverse direction to release the implant can be reduced. This can reduce the keyway angle which can make the transition of the clamshells from the respective first segment to the respective second segment smoother. This can improve the patient's perception of the retracting process.

According to an embodiment of the proposed solution, the keyways of the at least one first groove and the at least one second groove can be spaced apart from each other along the longitudinal direction. Thus, when starting in the carrying configuration both clamshells can be retracted along the longitudinal direction, whereby each of the clamshells are moved along the transverse direction at different positions along the longitudinal direction. This enables to adjust a transverse movement of the bottom clamshell relative to a transverse movement of the top clamshell in order to improve the release process.

According to an embodiment of the proposed solution, the keyway of the second groove is spaced apart from the keyway of the first groove along the longitudinal direction toward a distal end of the handle distal to the end against which the implant abuts in the carrying configuration.

The amount both keyways are spaced apart define a slippage amount by which the top clamshell can be retracted before entering the keyway the top clamshell is engaged with. Thus, the longitudinal distance between an end of the first keyway facing the proximal end of the handle and an end of the second keyway facing the proximal end of the handle can correspond to the slippage amount of the clamshells. According to this embodiment, the top clamshell can enter the keyway of the second groove when retracted by the slippage amount along the longitudinal direction toward the distal end. Starting from a fully protruded position both clamshells, i.e. the carrying configuration, the top clamshell can be retracted by the slippage amount without moving along the transverse direction.

The position of the clamshells in which the top clamshell is retracted by the slippage amount and the bottom clamshell is fully protruding can define a release configuration of the clamshells. In the release configuration the clamshells are configured to hold the implant and to move along the transverse direction if further retracted along the longitudinal direction. The first groove can be formed such that the bottom clamshell is moved along the transverse direction as soon as it is retracted from the fully protruded position.

Naturally the role of top and bottom clamshell can be interchanged. Also, there can be more than one first groove and more than just one second groove. In particular, there can be two first grooves in which the bottom clamshell is slidable and two second grooves in which the top clamshell is slidable.

According to an embodiment of the proposed solution, at least one of the clamshells can comprise a tapered tip segment. At least in the carrying configuration the tip segment faces away from the proximal end of the handle. In the retracted configuration the tip segment of the clamshells can be within the through-hole or outside the through-hole facing away from the proximal end of the handle.

The tapered tip segment can reduce force for inserting the implantation tool via an incision into a tissue. This can improve the perception of the insertion procedure. The tip segment may also be called tip.

The tip can be tapered with respect to the transverse direction and/or a further direction perpendicular to the transverse direction and the longitudinal direction. This can further reduce the force needed for insertion. Naturally, both clamshells can comprise a tapered tip segment.

The tip of at least one of the clamshells can be a part of a head including the broadest section of the clamshell. The head can extend along the transverse direction or the further direction above a bar segment of the clamshell. The bar segment can follow the head along the longitudinal direction. Thus, the head with the tip can be configured to form a subcutaneous pocket with a width corresponding to the broadest section of the clamshell, i.e. width of the head, while the bar segment of the clamshell is less wide. This can reduce material needed for the at least one clamshell which can reduce friction between said clamshell and a tissue in which the clamshells can be inserted. Additionally, the overall size of the implantation tool can be improved which can also improve the perception of the implantation tool by the patient. Also, less material can reduce production costs.

According to an embodiment of the proposed solution, the tip of one of the clamshells can be formed with a locking protrusion engaging with a locking recess of the other of the clamshells in the carrying configuration. Hence, the clamshells can be secured in the carrying configuration in order to avoid unintentional separation of both clamshells which might lead to unintentional releasing the medical implant. This can improve operating the proposed implantation tool.

According to an embodiment of the proposed solution, the locking protrusion and the locking recess can extend along the longitudinal direction. Hereby, in the carrying configuration the clamshells can be secured against any movement except of sliding along the longitudinal direction.

According to an embodiment of the proposed solution, in the carrying configuration the operating member may be locked or unlocked. In the locked carrying configuration, the clamshells are secured against movement in all directions. In the unlocked carrying configuration, the clamshells can be slid longitudinally.

According to an embodiment of the proposed solution, the locking recess can be formed in a segment with a sloped ramp. A surface of the ramp can face a segment of the other clamshell on which the locking protrusion is formed. This can enable smoothly moving the locking protrusion out and into the locking recess.

According to an embodiment of the proposed solution, a length of the locking protrusion along the longitudinal direction can correspond to the slippage amount. Thus, starting in the carrying configuration the clamshells can be secured during retraction until the release configuration is reached. In the release configuration the locking protrusion is not within the locking recess enabling movement of the clamshells along the transverse direction when the clamshells are further retracted. Thus, a probability of errors due to releasing the implant unintentionally because of external forces separating the clamshells in positions different from that defined by the grooves can be reduced.

According to an embodiment of the proposed solution, each of the clamshells can comprise at least one front tab via which they are slidably engaged with the respective groove of the handle. That is, the bottom clamshell may have one or more front tabs by which the bottom clamshell engages the at least one first groove. Analogously, the top clamshell may have one or more front tabs by which the top clamshell engages the at least one second groove. The tabs can provide a simple way to slidably engage the clamshells with the respective grooves.

According to an embodiment of the proposed solution, each of the clamshells can comprise at least one rear tab via which they are engaged with grooves of the operating member. Thus, the operating member can be used to retract the clamshells by retracting the operating member relative to the handle along the longitudinal direction. This means, that a retracting force introduced into said operating member can be introduced into clamshells via the engaged rear tabs of the clamshells. This can enable a comfortable way to retract the clamshells from the carrying configuration into the release configuration and into the retracted configuration.

According to an embodiment of the proposed solution, the operating member can be formed with a locking tab engaging a L-shaped locking groove formed in the handle. In the locked configuration of the operating member the locking tab can be located in a first part of the L-shaped locking groove. In said first part retracting the operating member along the longitudinal direction is blocked but rotation around the longitudinal direction is not blocked. Thus, via rotation of the operating member the locking tab can be brought into a second part of the locking groove in which the tab can slide along the longitudinal direction. According to this embodiment, the operating member is in the locked configuration if the locking tab is in the first part of the locking groove and the operation member is in the unlocked configuration if the locking tab is in the second part of the locking groove.

According to an embodiment of the proposed solution, the first part of the locking groove can extend circumferentially on the surface of the handle facing the through-hole. The second part of the locking groove can extend longitudinal along the surface of the handle facing the through-hole.

According to an embodiment of the proposed solution, while retracting the operating member the locking tab can leave the locking groove. Alternatively, the locking groove is formed to enable the locking tab to stay engaged when the clamshells are slid between the carrying configuration and the retracted configuration via retracting the operating member.

According to an embodiment of the proposed solution, the operating member can comprise a groove engaged with the at least one rear tab of the top clamshell. Said groove can extend circumferentially on a surface of the operating member. The surface of the operating member can extend on an outer or inner surface of the operating member, whereby outer and inner relates to a direction transvers to the longitudinal direction. Thus, if the operating member is rotated around the longitudinal axis, the at least one rear tab of the top clamshell slides within the groove of the operating member around the longitudinal direction. According to this embodiment, although engaged with the rear tab of the top clamshell the operating member can be rotated. As soon as the operating member is retracted also the top clamshell is retracted.

According to an embodiment of the proposed solution, the operating member can comprise a L-shaped further groove engaged with the at least one rear tab of the bottom clamshell. Accordingly, the further groove can comprise two parts extending in two different directions. Thus, the at least one rear tab of the bottom clamshell can slide in the two different directions within the further groove of the operating member. Considering the limited extension of the two parts of the further groove the operating member can be moved in the two different directions without introducing a force into the bottom clamshell. This means, the extension of the L-shaped further groove defines a slack of the operating member in which retracting/rotating the operating member does not cause the bottom clamshell to be moved. If the operating member is moved beyond the slack, a force can be introduced into the bottom clamshell, in particular the bottom clamshell can be retracted.

According to an embodiment of the proposed solution, the length of the locking protrusion corresponds to both a longitudinal spacing offset of the keyways as well as the L-shaped further groove on the operating member.

According to an embodiment of the proposed solution, the length of the locking protrusion along the longitudinal direction corresponds to a slippage amount defined by said at least one first groove and the at least one second groove, and/or by the amount both keyways are spaced apart.

According to an embodiment of the proposed solution, said further groove can comprise a circumferential part and a longitudinal part. If the at least one rear tab of the bottom clamshell is in the circumferential part of the further groove and the operating member is rotated around the longitudinal axis the at least one rear tab of the bottom clamshell can slide within the circumferential part of the further groove of the operating member. If the at least one rear tab of the bottom clamshell is in the longitudinal part of the further groove and the operating member is moved along the longitudinal axis, the at least one rear tab of the bottom clamshell can slide within the longitudinal part of the further groove of the operating member. Thus, although engaged with the at least one rear tab of the bottom clamshell the operating member can be rotated and retracted within the slack.

According to an embodiment of the proposed solution in which the top clamshell is engaged with the operating member via a circumferential groove and the bottom clamshell is engaged with the operating member via a L-shaped groove, the top clamshell can be retracted via the operating member from the carrying configuration while the bottom clamshell is only retracted if the operating member is retracted beyond the slippage amount. Thereby, via the further groove the slippage amount can be defined. Considering an embodiment with the locking protrusion and the locking recess the length of the longitudinal part of the further groove can correspond to the length of the locking protrusion. Thus, starting in the carrying configuration the top clamshell can be retracted via the operating member by the slippage amount in order to retract the locking protrusion out of the locking recess.

According to an embodiment of the proposed solution, the operating member can comprise a center rod extending along the longitudinal direction within the through-hole of the handle, said rod can comprise the groove and the further groove of the operating member. When properly installed, the center rod can be placed on a central axis of the through-hole aligning with the longitudinal direction. Thus, the center rod can be placed radially inward of the clamshells. Accordingly, the clamshells can be placed radially between the center rod and the handle.

Thereby, the center rod can prevent the clamshells from moving toward the central axis of the through-hole, which could move the clamshells out of contact with the at least one first and at least one second groove of the handle. This allows the center rod to secure the clamshells in said handle grooves.

According to an embodiment of the proposed solution, the center rod can comprise the groove and the further groove of the operating member on an outer surface facing the clamshells.

According to an embodiment of the proposed solution the center rod can comprise the at least one locking tab preventing the center rod from being retracted if the operating member is in the locking configuration. The locking tab can protrude radially outward on a surface of the center rod. Thereby, the locking groove can be formed on the surface of the handle facing the through-hole.

According to an embodiment of the proposed solution, the locking tab can be formed at an end of the center rod facing the proximal end of the handle. Thus, the second part of the locking groove can extend on the surface of the handle within the through-hole such that the locking tab stays engaged while retracting the clamshells from the carrying configuration via the release configuration into the retracted configuration. In such an embodiment, the locking tab enters the second part of the locking groove if the operating member is retracted from a position in which the clamshells are in the carrying configuration. When the locking tab is engaged with the second part of the locking groove rotation of the the operating member can be blocked. This can ease smooth operation of the tool.

According to an embodiment of the proposed solution, the operating member can comprise a ring and a knob for operating the operating member connected with the ring. The knob can be ergonomically formed to improve operation of the implanting tool. During manufacturing the ring can be engaged with the clamshells and afterwards connected to the knob. This can improve the manufacturing process.

According to an embodiment of the proposed solution, the handle can be ergonomically shaped to improve ergonomics.

According to an embodiment of the proposed solution, the handle can comprise at least a flat section along a circumference of the handle. This can reduce a tendency of the proposed implantation tool to roll when placed on a flat surface.

Another aspect of the proposed solution relates to a system. The proposed system comprises at least:
- an implantation tool according to the proposed solution; and
- a medical implant arranged between the top clamshell and the bottom clamshell in the carrying configuration.

In particular, said medical device is an implantable cardiac monitoring device.

Yet another aspect of the proposed solution relates to a method for implanting a medical implant. The proposed method at least comprises:
- providing an implantation tool according to the proposed solution, said implantation tool having a medical implant arranged between the top clamshell and the bottom clamshell in the carrying configuration;
- making an incision in the skin of the patient;
- inserting the clamshells of the implantation tool through the incision into the tissue of the patient, thereby defining a pocket in the tissue under the skin of the patient for insertion of the medical implant;
- unlocking the implantation tool by rotating the operating member from a locked into an unlocked configuration; and
- retracting the operating member while holding the handle in a fixed position in order to release the medical implant into the pocket.

According to the proposed method, a medical device, particularly an implantable cardiac monitoring device, can be implanted using only a relatively small number of steps (particularly, five steps, namely: 1. Make incision, 2. Insert clamshell to define pocket, 3. Rotate operating member to unlock the same, 4. Retract operating member while holding handle in place for releasing the medical device in pocket, 5. Close wound. Thereby, the proposed implantation not only offers an implantation procedure with such a small number of steps but also reduces size of the clamshells as well as the friction of the clamshells on the patient's tissue. Thus, perception of the tool itself as well as of the implantation can be improved.

Explanations of particular embodiments and advantages of the implantation tool apply analogously to the claimed system and method.

Other features which are considered as characteristic for the invention are set forth in the appended claims. Although the invention is illustrated and described herein as embodied in a tool and method for subcutaneous device insertion, it is nevertheless not intended to be limited to the details shown, since various modifications and structural changes may be made therein without departing from the spirit of the invention and within the scope and range of equivalents of the claims.

The construction and method of operation of the invention, however, together with additional objects and advantages thereof will be best understood from the following description of specific embodiments when read in connection with the accompanying drawings. Herein,
- Fig. 1: shows a perspective view of an embodiment of the proposed implantation tool;
- Figs. 2A, 2B: show a detailed view of the operating member of a implantation tool in a locked and an unlocked configuration;
- Fig. 3: shows another perspective view of the embodiment of Fig. 1 in an unassembled state;
- Fig. 4: shows a tip section of clamshells of the embodiment shown in Fig. 1;
- Fig. 5: shows a longitudinal section of a proximal end of a handle of the embodiment shown in Fig. 1;
- Fig. 6: shows the clamshells of the embodiment shown in Fig. 1 arranged around a center rod;
- Figs. 7A to 7C: show side views of a tip section of clamshells and operating members in different configurations;
- Fig. 8: shows a side view of an operating member and an unassembled bottom clamshell;
- Figs. 9A, 9B: show perspective views of the proposed system; and
- Fig. 10: shows a sequence of steps according to the proposed method.

Subsequently, embodiments of the proposed solution shall be described in detail with reference to the drawings. In the drawings, like reference numerals designate like structural elements.

It is to be noted that the embodiments are not limiting for the proposed solution, but merely represent illustrative examples.

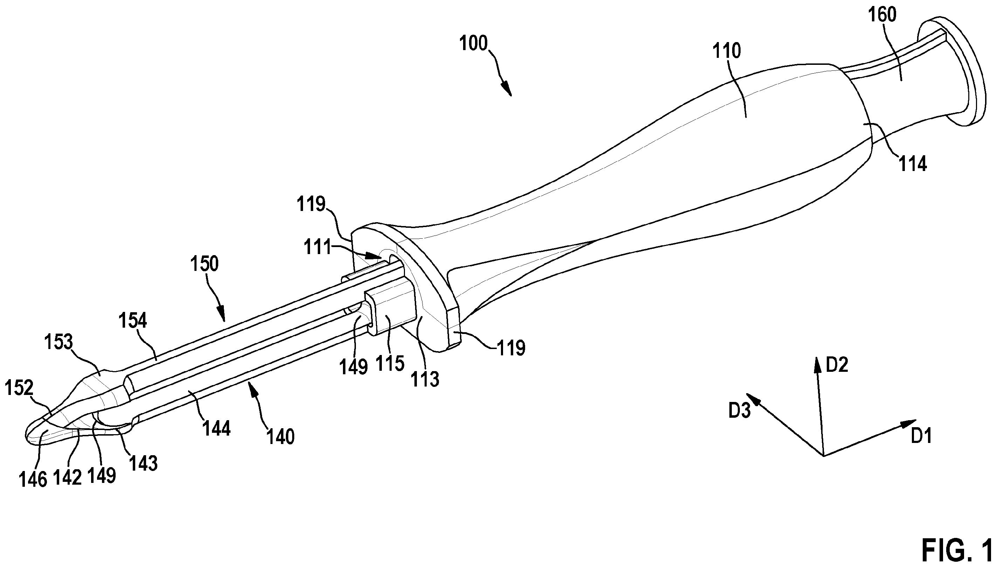

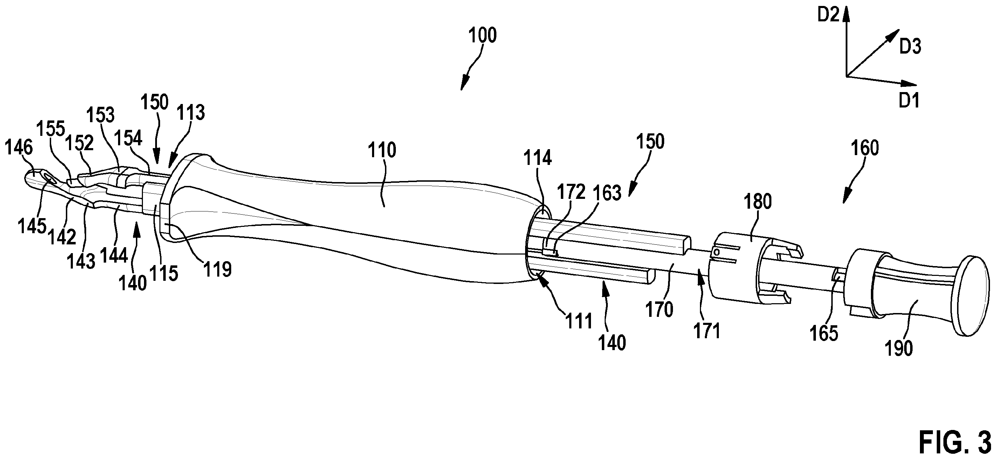

Referring now to the figures of the drawing in detail and first, particularly, to Figs. 1 and 3 thereof, there are shown the basic components of an exemplary embodiment of an implantation tool 100 for implanting a medical device 200 subcutaneously according to the present invention. As indicated in Figs. 1 and 3 the implantation tool 100 for implanting a medical implant 200 subcutaneously comprises a handle 110 extending along a longitudinal direction D1, whereby said handle 110 is formed with a through-hole 111, extending along the longitudinal direction D1. The handle 110 is also formed with at least one first groove 120 and at least one second groove 130, said grooves 120, 130 extending on a surface 112 of the handle 110 facing the through-hole 111. A bottom clamshell 140 is slidably mounted in said at least one first groove 120 and a top clamshell 150 is slidably mounted in said at least one second groove 130. Said grooves 120, 130 are formed to define a carrying configuration of said clamshells 140, 150, in which said clamshells 140, 150 protrude from the handle 110 to hold a medical implant 200 abutting a proximal end 113 of the handle 110 which is proximal to the clamshells 140, 150. This carrying configuration is shown in Fig. 1. Said grooves 120, 130 are also formed such that said clamshells 140, 150, when retracted from the carrying configuration toward the handle 110 to a retracted configuration, are shifted away from each other along a transverse direction D2 transverse to the longitudinal direction D1 to release the medical implant 200 from the implantation tool 100. Furthermore, the implantation tool 100 comprises an operating member 160 engaged with said clamshells 140, 150 whereby via a rotation of said operating member 160 relative to said handle 110 said operating member 160 is switchable between a locked configuration and an unlocked configuration. Said operating member 160 is configured to block sliding of said clamshells 140, 150 along said grooves 120, 130 in the locked configuration and to allow sliding of said clamshells 140, 150 via the operating member 160 in the unlocked configuration.

The top clamshell 150 as well as the bottom clamshell 140 each comprise a head 143, 153 including the broadest section of the clamshells 140, 150 and a segment 122, 124, 132, 134, 144, 154 which is adjacent to the head 143, 153 along the longitudinal direction D1. Thereby, the head 143, 153 extends along a further direction D3 transverse to the longitudinal direction D1 above the bar segment 144, 154 of the clamshells 140, 150. The head 153 of the top clamshell 150 as well as the head 143 of the bottom clamshell 140 both comprise a tapered tip 142,152 configured to form a subcutaneous pocket with a width corresponding to the broadest section of the clamshells 140, 150, i.e. width of the heads 143, 153. The bar segments 144, 154 of the clamshells 140, 150 are less wide.

The tip 152 of the top clamshell 150 comprises a locking protrusion 155 which is configured to engage with a locking recess 145 which is formed on the tip 142 of the bottom clamshell 140 when the clamshells 140, 150 are in the carrying configuration. Both the locking protrusion 155 and the locking recess 145 extend along the longitudinal direction D1 in order to secure the clamshells 140, 150 against movement transverse to the longitudinal direction D1 if locking protrusion 155 and locking recess 145 are engaged.

The head 143 of the bottom clamshell 140 also comprises a sloped ramp 146 in which the locking recess 145 of the bottom clamshell 140 is formed enabling smoothly moving the locking protrusion 155 out and into the locking recess 145.

The clamshells 140, 150 are configured to hold a medical implant 200 in between both clamshells 140, 150. Accordingly, the medical implant 200 will be placed with the top clamshell 150 on top and the bottom clamshell 140 under the medical implant 200. To hold the medical implant 200 in place the bottom clamshell 140 comprises an implant contour 149 at the boundary of the head 143 to the bar segment 144. The implant contour 149 is formed to rest against the medical implant 200 if it is hold between the clamshells 140, 150 in the carrying configuration. Naturally, also the top clamshell 150 can be formed with such implant contours 149. Furthermore, implant contours 149 can also be formed on an end of the bar segments 144, 154 adjacent to the handle 110.

The handle 110 of the implantation tool 100 also comprises an abut section 115 on the proximal end 113 proximal to the tips 142, 152 of the clamshells 140, 150. This abut section 115 is formed such that the medical implant 200 rest against it and thus is not retraced if the clamshells 140, 150 are retracted form the carrying configuration along the longitudinal direction D1 toward the proximal end 113 of the handle 110. Additionally, the handle 110 also comprises two flat sections 119 on opposite sides of the handle 110. Those flat sections 119 hinder the implantation tool 100 to roll on flat surfaces 112. Incidentally, the handle 110 is ergonomically formed to improve operation of the implantation tool 100.

At a distal end 114 of the handle 110 opposite to the proximal end 113 the operating member 160 is arranged via which the clamshells 140, 150 can be retracted.

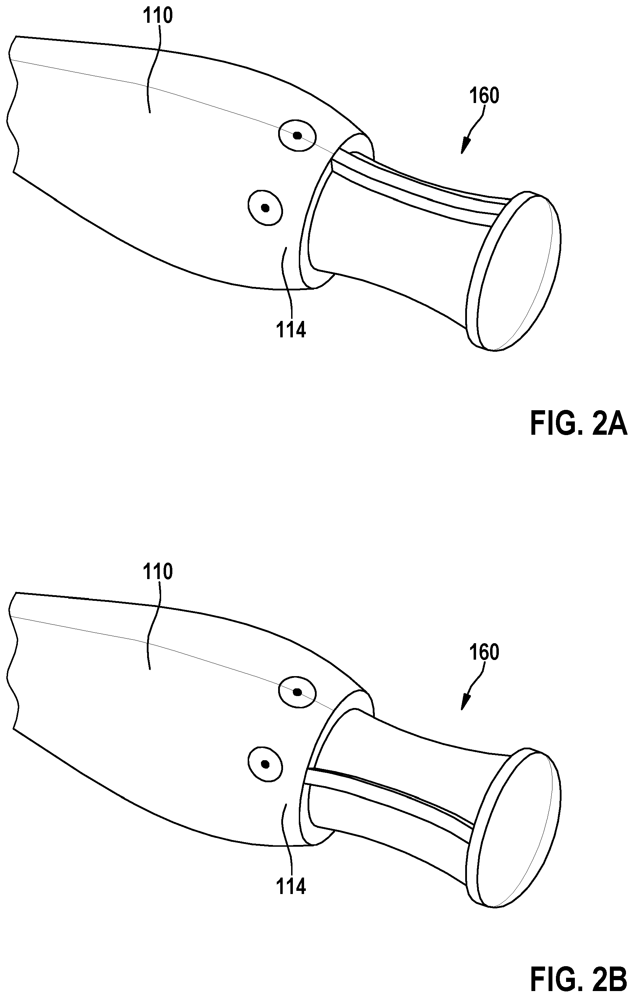

To lock the clamshells 140, 150 in the carrying configuration shown in Figure 1, the operating member 160 can be locked. In the locked configuration the operating member 160 cannot be retracted. To enable retracting the operating member 160 can be rotated from the locked configuration into the unlocked configuration. Such a change of operating member 160 configurations is shown in Figs. 2A and 2B.

As indicated by the line on the operating member 160 pointing to the closed lock Fig. 2A shows the operating member 160 in the locked configuration in which it is not retractable. Thus, the clamshells 140, 150 are not retractable. In Fig. 2B the operating member 160 is rotated such that the line on the operating member 160 points to the opened lock indicating that the operating member 160 is in the unlocked configuration.

In the embodiment shown in Fig. 1 locking of the operating member 160 is realized via a center rod 170 depicted in Fig. 3. Thereby, the operating member 160 consists of several elements, namely the center rod 170, a ring 180 and a knob 190. In an assembled state all elements 170, 180, 190 are connected to each other in a rotationally fixed manner. Thus, none of said elements 170, 180, 190 can be rotated relative to one of the other elements 170, 180, 190. The center rod 170 extends along the longitudinal direction D1 and is placed within the through-hole 111 of the handle 110 in the assembled state. When properly installed, the center rod 170 is placed on a central axis of the through-hole 111 aligning with the longitudinal direction D1. Thus, the center rod 170 is placed radially inward of the clamshells 140, 150. Accordingly, the clamshells 140, 150 are placed radially between the center rod 170 and the handle 110. Thereby, the center rod 170 prevents the clamshells 140, 150 from moving toward the central axis of the through-hole 111, which could move the clamshells 140, 150 out of contact with the at least one first groove 120 and the at least one second groove 130. Thus, the center rod 170 secures the clamshells 140, 150 in said handle 110 grooves 120, 130. To realize the locking and unlocking configuration the center rod 170 comprises two locking tabs 163 (only one shown here). The locking tabs 163 protrude radially outward on an outer surface 171 of the center rod 170 and are arranged on opposite sides of the center rod 170. In the assembled state each of the locking tabs 163 engage with one of two L-shaped locking grooves formed on the surface 112 of the handle 110 facing the through-hole 111. In the locked configuration each of the both locking tabs 163 are located in a first part of one of the L-shaped locking grooves. In said first part retracting the operating member 160 along the longitudinal direction D1 is blocked but rotation around the longitudinal direction D1 is not blocked. Thus, via rotation of the operating member 160 the locking tab 163 (formed at an end 172 of the center rod 170) can be brought into a second part of the locking groove in which the locking tab 163 can slide along the longitudinal direction D1. Accordingly, the operating member 160 is in the locked configuration if the locking tab 163 is in the first part of the locking groove and the operation member 160 is in the unlocked configuration if the locking tab 163 is in the second part of the locking groove. Furthermore, the center rod 170 comprises at least one groove 164, 165 on the outer surface 171 facing the clamshells 140, 150 via which the clamshells 140, 150 are engaged with the operating member 160.

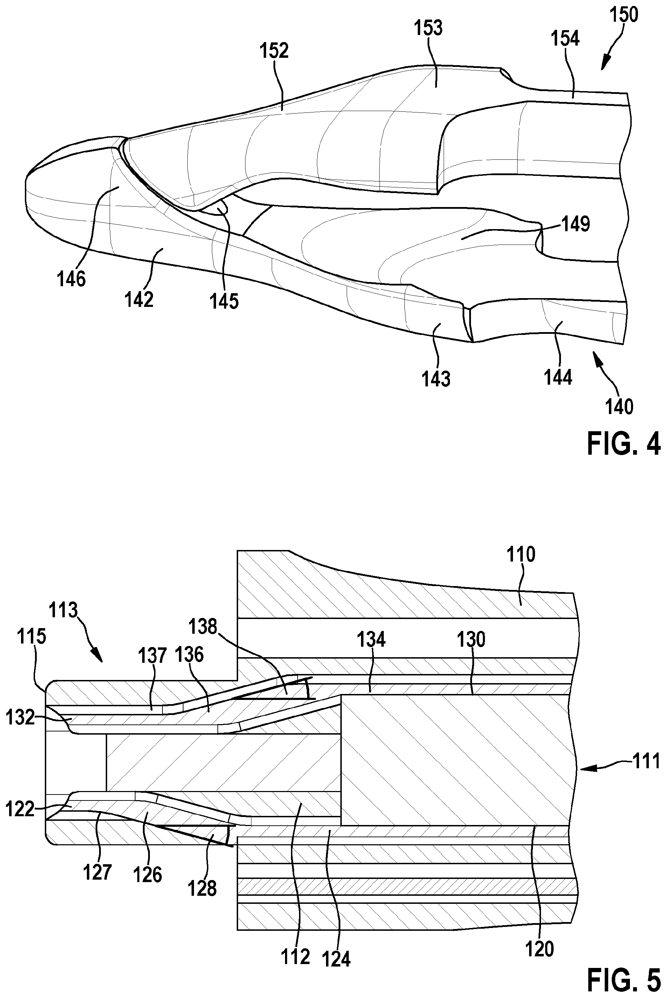

Details of the head 143, 153 of the bottom clamshell 140 and the top clamshell 150 shown in Figs. 1 and 3 are depicted in Fig. 4. In particular, it can be seen that surfaces of the clamshells 140, 150 intended to face the medical implant 200 hold between the clamshells 140, 150 are flat. In contrast, surfaces of the clamshells 140, 150 at the head 143, 153 are sloped. At the boundary the implant contour 149 is formed.

Fig. 5 shows a cross section through a part of the handle 110 including the proximal end 113. The surface 112 of the handle 110 facing the through-hole 111 is formed with the first and the second grooves 120, 130. Both grooves 120, 130 comprise two parallel segments 122, 124, 132, 134 spaced apart from each other along the transverse direction D2. Thus, the clamshells 140, 150 engaged with the first and second grooves 120,130 are moved along the transverse direction D2 when slid from the one of the two parallel segments 122, 132 to the other of the two segments 124, 134. The two parallel segments 122, 124, 132, 134 are connected by an angled keyway 126, 136 along which the respective clamshell 140, 150 engaged with the first or second groove 120, 130 slides from the first of the parallel segments 122, 132 into the second of the parallel segments 124, 134. Thus, the keyways 126, 136 extend along the longitudinal direction D1 and along the transverse direction D2 enclosing an angle 128, 138 with the longitudinal direction D1 (keyway angle). The keyways 126, 136 for the top clamshell 150 and for the bottom clamshell 140 are spaced apart from each other along the longitudinal direction D1. The amount both keyways 126, 136 are spaced apart define a slippage amount 151 by which the top clamshell 150 can be retracted before entering the second keyway 136. Thus, the longitudinal distance between an end 127 of the first keyway 126 facing the proximal end 113 of the handle 110 and an end 137 of the second keyway 136 facing the proximal end 113 of the handle 110 represent the slippage amount 151 of the clamshells 140, 150.

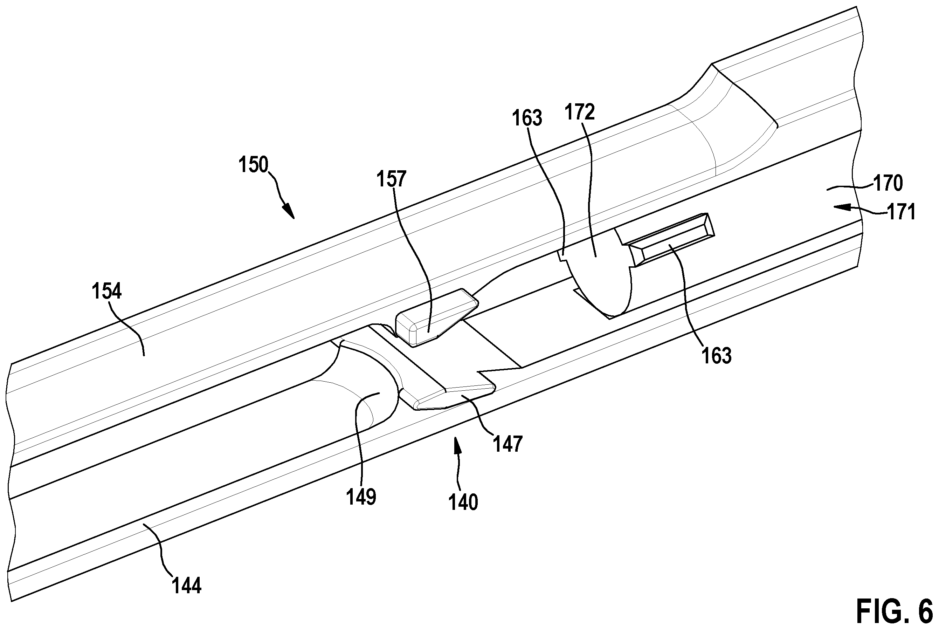

Although only one first and one second groove 120, 130 are shown in Fig. 5 the depicted embodiment is formed with a pair of two first and two second grooves 120, 130 arranged opposite to each other on the surface 112 of the handle 110 facing the through-hole 111. Engagement of the clamshells 140, 150 with the first and second grooves 120, 130 are realized via a pair of front tabs 147 on the bottom clamshell 140 and via a pair of front tabs 157 on the top clamshell 150. For each of the clamshells 140, 150 one of the two front tabs 147, 157 are shown in Fig. 6.

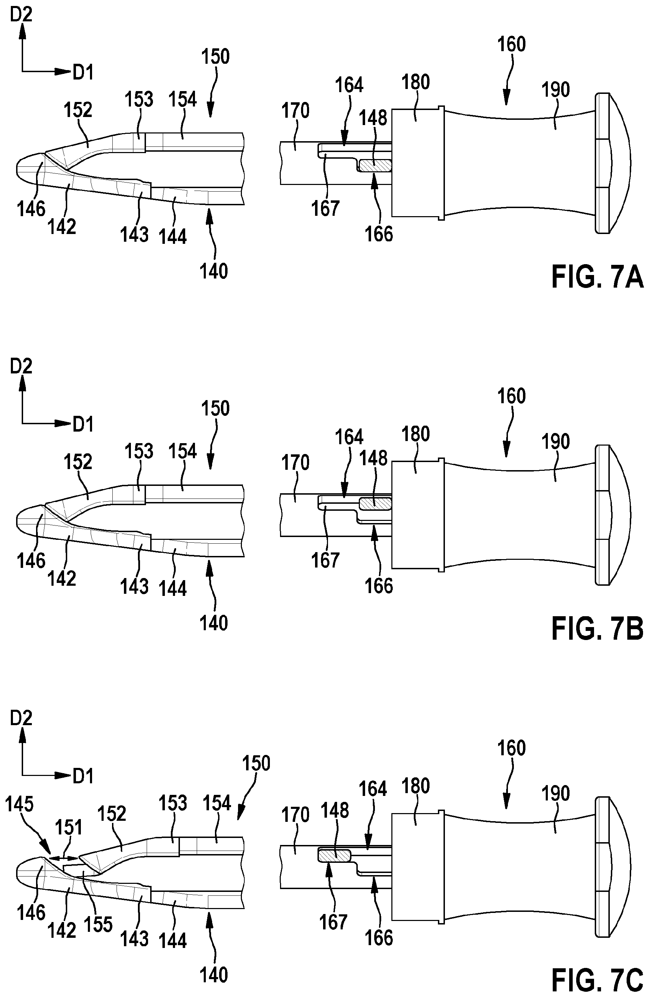

Figs. 7A, 7B and 7C show details about the engagement of the bottom clamshell 140 with the operating member 160 and the position of the clamshells 140, 150 in different configurations.

Here, Fig. 7A shows a side view of a part of the clamshells 140, 150 around the heads 143, 153 as well as a part of the operating member 160. The center rod 170 of the operating member 160 comprises a groove 165 not shown in Figs. 7A, 7B and 7C engaged with one rear tab 148 of the top clamshell 150. Said groove 165 extends circumferentially on the surface 171 of the center rod 170 on a side opposite to the further groove 164 depicted in Figs. 7A, 7B and 7C. The L-shaped further groove 164 is engaged with the rear tab 148 of the bottom clamshell 140. Due to the L-shape, the further groove 164 comprises two parts 166, 167 extending in two different directions, i.e. the longitudinal direction D1 and a circumferential direction. Thus, said further groove 164 comprise a circumferential part 166 and a longitudinal part 167. The extension of the L-shaped further groove 164 defines a slack of the operating member 160 in which retracting/rotating the operating member 160 does not cause the bottom clamshell 140 to be moved. If the operating member 160 is moved beyond the slack a force can be introduced into the bottom clamshell 140, in particular the bottom clamshell 140 can be retracted.

In Fig. 7A the clamshells 140, 150 are in the carrying configuration and the operating member 160 is in the locked configuration. The rear tab 148 of the bottom clamshell 140 is located in the circumferential part 166 of the further groove 164. As the operating member 160 is locked, no retraction is possible but the operating member 160 can be rotated around the longitudinal direction D1 into the unlocked configuration, which is shown in Fig. 7B.

In Fig. 7B, the rear tab 148 of the bottom clamshell 140 is located in the longitudinal part 167 of the further groove 164 at an end distal to the clamshells 140, 150. Accordingly, if the operating member 160 is retracted along the longitudinal direction D1, the rear tab 148 of the bottom clamshell 140 slides within the longitudinal part 167 of the further groove 164 until it rests on a contour of the further groove 164 proximal to the clamshells 140, 150. This position is shown in Fig. 7C.

Fig. 7C shows the configuration of the clamshells 140, 150 which can be reached by retracting the operating member 160 starting in the carrying configuration until the rear tab 148 of the bottom clamshells 140 rests on said contour of the further groove 164 proximal to the clamshells 140, 150. As the top clamshell 150 is engaged with the operating member 160 such that retracting the operating member 160 always retracts the top clamshell 150 the top clamshell 150 and the bottom clamshell 140 are spaced apart by the slippage amount 151 which is defined by the extension of the longitudinal part 167 of the further groove 164. If the operating member 160 is retracted further, both clamshells 140, 150 will be retracted as well. As stated above, the at least one first groove 120 and the at least one second groove 130 are formed to provide a movement of the clamshells 140, 150 along the transverse direction D2 if the operating member 160 is retracted further. Thus, the configuration of the clamshells 140, 150 in Fig. 7C represents a release configuration of the clamshells 140, 150 from which the medical implant 200 is released by further retracting the operating member 160.

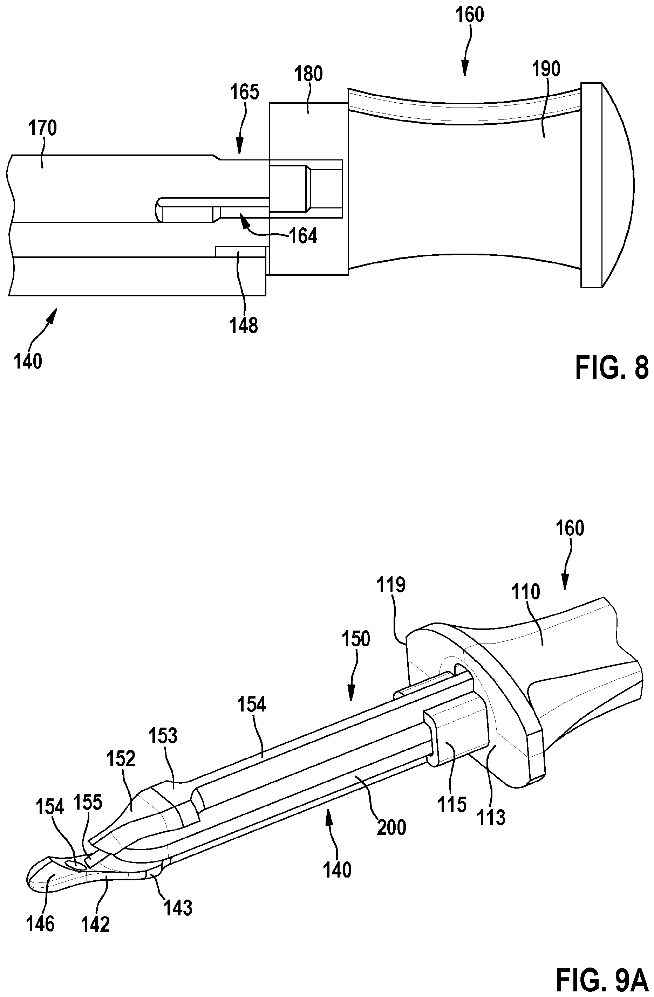

Fig. 8 shows another side view of the operating member 160 with the center rod 170. On two opposite sides the circumferential groove 165 for engaging the top clamshell 150 (not shown) and the L-shaped further groove 164 for engaging the bottom clamshell 140 are formed.

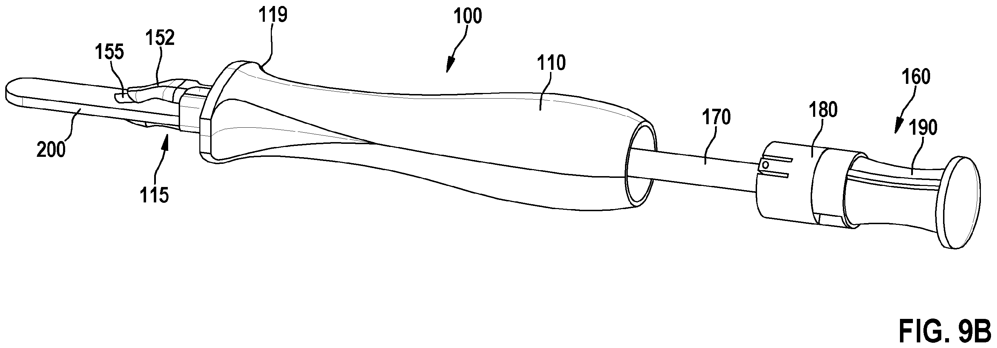

Figs. 9A and 9B show an embodiment of the proposed system comprising the proposed implantation tool 100 and a medical implant 200. In Fig. 9A the clamshells 140, 150 are in the release configuration. In Fig. 9B the clamshells 140, 150 are in the retracted configuration.

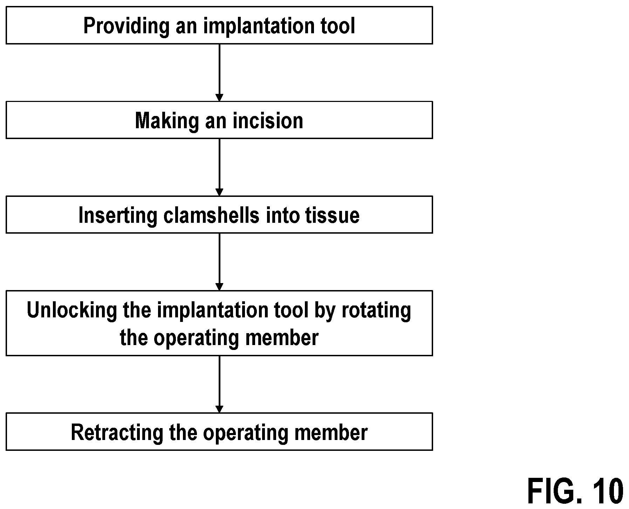

Fig. 10 shows a sequence of steps according to the proposed method. Thus, in a first step an implantation tool 100 according to the proposed solution is provided, said implantation tool 100 having a medical implant 200 arranged between the top clamshell 150 and the bottom clamshell 140 in the carrying configuration. In another step, an incision in the skin of the patient is made in order to insert the clamshells 140, 150 of the implantation tool 100 through the incision into the tissue of the patient. Thereby a pocket in the tissue under the skin of the patient for insertion of the medical implant 200 is defined. Subsequently, the implantation tool 100 is unlocked by rotating the operating member 160 from the locked into the unlocked configuration. Finally, the operating member 160 is retracted while holding the handle 110 in a fixed position in order to release the medical implant 200 into the pocket.

Thereby, retracting the operating member 160 causes the clamshells 140, 150 to be retracted from the carrying configuration to the retracted configuration. The retraction of the clamshells 140,150 comprises the following sub-steps: 1. Adjusting the clamshells 140, 150 into the release configuration which releases the engagement of locking protrusion 155 and locking recess 145 and which is reached by retracting the top clamshell 150 by the slippage amount 151 while the bottom clamshell 140 is not retracted; and simultaneously spacing the clamshells 140, 150 apart from each other while the clamshells 140, 150 are further retracted into the retracted configuration.

### Reference Numerals

- 100: implantation tool

- 110: handle
- 111: through-hole
- 112: surface
- 113: proximal end
- 114: distal end
- 115: abut section
- 119: flat section

- 120: first groove
- 122, 124: segment
- 126: first keyway
- 127: end
- 128: angle

- 130: second groove
- 132, 134: segment
- 136: second keyway
- 137: end
- 138: angle

- 140: bottom clamshell
- 142: tip
- 143: head
- 144: bar segment
- 145: locking recess
- 146: ramp
- 147: front tab
- 148: rear tab
- 149: implant contour

- 150: top clamshell
- 151: slippage amount
- 152: tip
- 153: head
- 154: bar segment
- 155: locking protrusion
- 157: front tab

- 160: operating member
- 163: locking tab
- 164, 165: groove
- 166,167: groove parts

- 170: center rod
- 171: outer surface
- 172: end

- 180: ring
- 190: knob

- 200: medical implant

- D1: longitudinal direction
- D2: transverse direction
- D3: further direction

## Claims

1. An implantation tool (100) for implanting a medical implant (200) subcutaneously, the implantation tool (100) comprising:
- a handle (110) extending along a longitudinal direction (D1), said handle (110) being formed with a through-hole (111), extending along the longitudinal direction (D1), and at least one first groove (120) and at least one second groove (130), said grooves (120, 130) extending on a surface (112) of the handle (110) facing the through-hole (111);
- a bottom clamshell (140) slidably mounted in said at least one first groove (120) and a top clamshell (150) slidably mounted in said at least one second groove (130), said grooves (120, 130) are formed to define a carrying configuration of said clamshells (140, 150), in which said clamshells (140, 150) protrude from the handle (110) to hold the medical implant (200) abutting an end (113) of the handle (110), and to assume to retract said clamshells (140, 150) from said carrying configuration toward the handle (110) into a retracted configuration, whereby said clamshells (140, 150) are shifted away from each other along a transverse direction (D2) transverse to the longitudinal direction (D1) for releasing the medical implant (200) from the implantation tool (100); and
- an operating member (160) engaged with said clamshells (140, 150) whereby via a rotation of said operating member (160) relative to said handle (110) said operating member (160) is switchable between a locked configuration and an unlocked configuration, said operating member (160) is configured to block sliding of said clamshells (140, 150) along said grooves (120, 130) in the locked configuration and to allow sliding of said clamshells (140, 150) via the operating member (160) in the unlocked configuration.

2. The implantation tool (100) according to claim 1, wherein the at least one first groove (120) and/or the at least one second groove (130) comprise two parallel segments (122, 124, 132, 134) spaced apart from each other along the transverse direction (D2) transverse to the longitudinal direction (D1).

3. The implantation tool (100) according to claim 2, wherein the two parallel segments (122, 124, 132, 134) are connected by an angled keyway (126, 136) formed to enable an engaged clamshell (140, 150) to slide from the first of the parallel segments (122, 124) into the second of the parallel segments (122, 124).

4. The implantation tool (100) according to claim 3, wherein the at least one first groove (120) and the at least one second groove (130) comprise keyways (126, 136) and the keyways (126, 136) of the at least one first groove (120) and the at least one second groove (130) are spaced apart along the longitudinal direction (D1).

5. The implantation tool (100) according to claim 4, wherein the keyway (136) of the at least one second groove (130) is spaced apart by a slippage amount (151) from the keyway (126) of the at least one first groove (120) along the longitudinal direction (D1) toward an distal end (114) of the handle (110) distal to the proximal end (113) on which the implant (200) abuts in the carrying configuration.

6. The implantation tool (100) according to claim 1, wherein at least one of said clamshells (140, 150) comprise a tapered tip (142, 152).

7. The implantation tool (100) according to claim 1, wherein a tip (142, 152) of one of said clamshells (140, 150) is formed with a locking protrusion (155) engaging in the carrying configuration with a locking recess (145) of the other of said clamshells (140, 150).

8. The implantation tool (100) according to claim 7, wherein a length of the locking protrusion (155) along the longitudinal direction (D1) corresponds to a slippage amount (151) defined by said at least one first groove (120) and the at least one second groove (130) and/or by the amount both keyways (126, 136) are spaced apart.

9. The implantation tool (100) according to claim 1, wherein said operating member (160) comprises a groove (165) engaged with at least one rear tab of the top clamshell (150).

10. The implantation tool (100) according to claim 9, wherein said operating member (160) comprises a L-shaped further groove (164) engaged with at least one rear tab (148) of the bottom clamshell (140).

11. The implantation tool (100) according to claim 10, wherein said operating member (160) is formed with at least one locking tab (163) engaging a L-shaped locking groove formed in the handle (110).

12. The implantation tool (100) according to claim 11, wherein said operating member (160) comprises a center rod (170) extending along the longitudinal direction (D1) within the through-hole (111) of the handle (110), said center rod (170) comprises the grooves (164, 165) of the operating member (160).

13. The implantation tool (100) according to claim 12, wherein the at least one locking tab (163) is formed on the center rod (170) preventing the center rod (170) from being retracted if the operating member (160) is in the locking configuration.

14. A system, comprising:
- an implantation tool (100) according to claim 1; and
- a medical implant (200) arranged between the top clamshell (150) and the bottom clamshell (140) in the carrying configuration.

15. A method, for implanting a medical implant (200), comprising:
- providing an implantation tool (100) according to claim 1, said implantation tool (100) having a medical implant (200) arranged between the top clamshell (150) and the bottom clamshell (140) in the carrying configuration;
- making an incision in the skin of the patient;
- inserting the clamshells (140, 150) of the implantation tool (100) through the incision into the tissue of the patient, thereby defining a pocket in the tissue under the skin of the patient for insertion of the medical implant (200);
- unlocking the implantation tool (100) by rotating the operating member (160) from the locked into the unlocked configuration; and
- retracting the operating member (160) while holding the handle (110) in a fixed position in order to release the medical implant (200) into the pocket.
